(19) Europäisches Patentamt
European Patent Office
Office européen des brevets

(11) Veröffentlichungsnummer: **0 346 684 A1**

# (12) EUROPÄISCHE PATENTANMELDUNG

(21) Anmeldenummer: **89109809.7**

(51) Int. Cl.⁴: **C07D 271/04 , A61K 31/41**

(22) Anmeldetag: **31.05.89**

Patentansprüche für folgende Vertragsstaaten: ES + GR.

(30) Priorität: **11.06.88 DE 3819914**

(43) Veröffentlichungstag der Anmeldung: **20.12.89 Patentblatt 89/51**

(84) Benannte Vertragsstaaten: **AT BE CH DE ES FR GB GR IT LI NL SE**

(71) Anmelder: **CASSELLA Aktiengesellschaft**
**Hanauer Landstrasse 526**
**D-6000 Frankfurt am Main 61(DE)**

(72) Erfinder: **Schönafinger, Karl, Dr.**
**Holunderweg 8**
**D-8755 Alzenau(DE)**
Erfinder: **Beyerle, Rudi, Dr.**
**An der Pfaffenmauer 44**
**D-6000 Frankfurt/Main 60(DE)**
Erfinder: **Bohn, Helmut, Dr.**
**Kranzbergring 11**
**D-6369 Schöneck 1(DE)**
Erfinder: **Just, Melitta, Dr.**
**Weimarer Strasse 2**
**D-6369 Nidderau 2(DE)**

(74) Vertreter: **Urbach, Hans-Georg, Dr. et al**
**Hanauer Landstrasse 526**
**D-6000 Frankfurt am Main 61(DE)**

## (54) Substituierte 3-Aminosydnonimine, Verfahren zu ihrer Herstellung und ihre Verwendung.

(57) Substituierte 3-Aminosydnonimine der Formel I

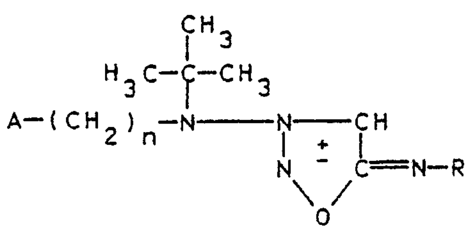

(I)

und ihre pharmakologisch annehmbaren Säureadditionssalze, worin
A z.B. Dimethylamino oder Hydroxy;
R Wasserstoff oder den Rest $-COR^1$,
$R^1$ z.B. einen aliphatischen Rest mit 1 bis 4 C-Atomen,
n die Zahl 2 oder 3
bedeuten, werden durch Cyclisierung einer Verbindung der Formel II

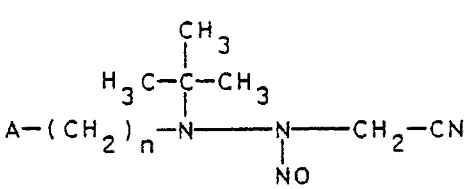

(II)

EP 0 346 684 A1

und gegebenenfalls anschließende Acylierung hergestellt und besitzen wertvolle pharmakologische Eigenschaften.

## Substituierte 3-Aminosydnonimine, Verfahren zu ihrer Herstellung und ihre Verwendung

Die Erfindung betrifft pharmakologisch wirksame substituierte 3-Aminosydnonimine der allgemeinen Formel I

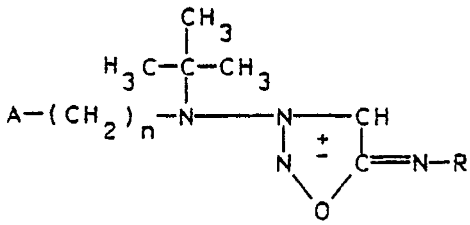

(I)

und ihre pharmakologisch annehmbaren Säureadditionssalze, worin
A Hydroxy; Alkoxy mit 1 bis 4 C-Atomen; Dialkylamino mit insgesamt 2 bis 8 C-Atomen; Morpholino; Piperidino; Pyrrolidin-1-yl;
R Wasserstoff oder den Rest $-COR^1$;
$R^1$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 2 oder 3
bedeuten.

Die Erfindung betrifft ferner ein Verfahren zur Herstellung der erfindungsgemäßen Verbindungen und ihre Verwendung.

Für n ist die Zahl 2 bevorzugt.

Aliphatische Reste, Alkylreste und Alkoxyreste können geradkettig oder verzweigt sein. Dies gilt auch, wenn sie als Substituenten anderer Reste, z.B. als Substituenten für Arylreste, oder in Verbindung mit anderen Resten auftreten, z.B. als Phenalkyl oder als Alkoxycarbonyl.

Beispiele für die für A stehende Dialkylaminogruppe mit insgesamt 2 bis 8 C-Atomen sind N,N-Dimethyl-, N,N-Diethyl-, N,N-Dipropyl- oder N,N-Dibutyl-amino; N,N-Di-isopropylamino; N-Methyl-N-ethyl-amino; N-Methyl-N-isopropyl-amino; N-Ethyl-N-sec-butyl-amino; N-Ethyl-N-propyl-amino; N-Propyl-N-butyl-amino.

Bevorzugte Bedeutungen von A sind: Hydroxy, Methoxy, Ethoxy, Isopropoxy, Dimethylamino, Diethylamino, Di-n-propylamino, Diisopropylamino, Morpholino.

Als für $R^1$ stehende aliphatische Reste kommen insbesondere Alkylreste mit 1 bis 4 C-Atomen in Betracht. Als für $R^1$ stehende aliphatische Reste, die durch Alkoxy mit 1 bis 3 C-Atomen substituiert sind, ist insbesondere der Methoxymethylrest zu nennen. Als für $R^1$ stehende cycloaliphatische Reste kommen vor allem Cycloalkylreste mit 5 bis 7 C-Atomen, insbesondere Cyclopentyl, und bevorzugt Cyclohexyl, in Betracht. Als für $R^1$ stehender bicycloaliphatischer Rest kommt insbesondere das 2,6,6-Trimethylbicyclo-(3.1.1)heptan-3-yl (=Pinanyl-3) in Betracht.

Als für $R^1$ stehender tricycloaliphatischer Rest kommt insbesondere das Tricyclo(3.3.1.1$^{3,7}$)decan-1-yl (=Adamantanyl) in Betracht. Als für $R^1$ stehende Alkoxyreste kommen insbesondere solche mit 1 bis 4 C-Atomen, vor allem Methoxy- und Ethoxy-reste in Betracht. Als für $R^1$ stehende Alkoxycarbonylreste kommen insbesondere solche mit insgesamt 2 bis 4 C-Atomen, vor allem der Ethoxycarbonylrest in Betracht. Als für $R^1$ stehende Arylreste sind z.B. $\alpha$- oder $\beta$-Naphthylreste, insbesondere aber der Phenylrest, zu nennen. Als für $R^1$ stehende Aryloxyreste sind z.B. $\alpha$- oder $\beta$-Naphthoxyreste, insbesondere aber der Phenoxyrest, zu nennen. Die für $R^1$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl-4-nitrophenyl. Als Halogensubstituenten für die Arylreste kommen z.B. Chlor und Bromatome in Betracht. Als für $R^1$ stehende substituierte Arylreste sind insbesondere zu nennen: Methylphenyl (= Tolyl), Nitrophenyl und Chlorophenyl, insbesondere 4-Nitrophenyl und 4-Chlorophenyl.

Für $R^1$ sind bevorzugt: Alkylreste mit 1 bis 4 C-Atomen, Alkoxyreste mit 1 bis 4 C-Atomen, Cycloalkylreste mit 5 oder 6 C-Atomen. Ganz besonders bevorzugt sind: Methyl, Ethyl, Propyl, Isopropyl, tert-Butyl, Cyclohexyl, Methoxy, Ethoxy, Isopropoxy, Butoxy, Phenyl.

Bevorzugte Bedeutungen von R sind Wasserstoff und $-COR^1$ mit den vorstehend angegebenen bevorzugten und insbesondere mit den angegebenen besonders bevorzugten Bedeutungen von $R^1$.

Eine Verbindung der allgemeinen Formel I kann dadurch hergestellt werden, daß eine Verbindung der allgemeinen Formel II

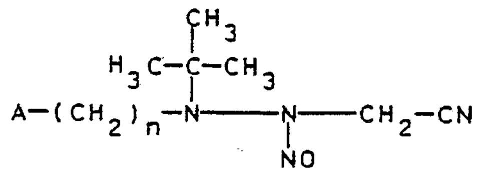

(II)

worin A und n die bereits genannten Bedeutungen besitzen, zu einer Verbindung der allgemeinen Formel Ia

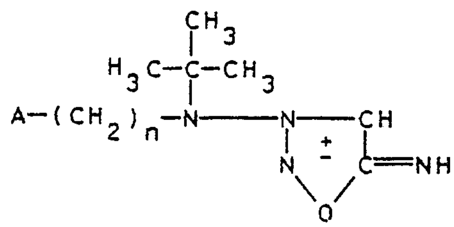

(Ia)

cyclisiert wird und daß diese oder ein Säureadditionssalz davon für den Fall, daß eine Verbindung der Formel I mit R = $-COR^1$ hergestellt werden soll, mit einem Acylierungsmittel, das den Rest $-COR^1$ einführt, acyliert wird und die so erhaltene Verbindung gegebenenfalls in ein pharmakologisch annehmbares Säureadditionssalz überführt wird.

Die Cyclisierung der Verbindungen II zu den Verbindungen Ia wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert unter 3 einstellen, also z.B. starke Säuren, wie Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt. Von dem Cyclisierungsmittel kommt z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Besonders bequem ist die Verwendung von Chlorwasserstoff als Cyclisierungsmittel, der normalerweise bis zur Sättigung in den Reaktionsansatz eingeleitet wird. Bei der Cyclisierung wird normalerweise das entsprechende Säureadditionssalz der Verbindung Ia erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butylether, Tetrahydrofuran, 1,4-Dioxan, 1,2-Dimethoxyethan, Bis-β-methoxyethylether; Oligoethylen-glykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl-oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Di-methylcyclohexanon, Benzophenon, Acetophenon; aliphatische Kohlenwasserstoffe, wie z.B. Hexan, Heptan, niedrig-und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugsweise Essigsäureethylester-Methanol.

Die Verbindungen der Formel Ia stellen erfindungsgemäße Verbindungen der allgemeinen Formel I für den Fall dar, daß R = Wasserstoff ist.

Die Acylierung der Verbindung der Formel Ia, die auch in Form eines Säureadditionssalzes vorliegen kann, zur Einführung des Restes R = $-COR^1$ kann in an sich bekannter Weise mit einem geeigneten Acylierungsmittel der Formel III durchgeführt werden

$$X-\overset{\overset{\displaystyle O}{\|}}{C}-R^1 \quad (III)$$

worin X ein nukleophil abspaltbarer Rest darstellt.

In der Formel III bedeutet X z.B. insbesondere Halogen, vorzugsweise -Cl oder -Br; -OH; -O-Alkyl, insbesondere mit 1 bis 5 C-Atomen; -O-Aryl, wobei der Arylrest insbesondere ein Phenylrest ist, der auch mit Alkyl, insbesondere Methyl, und/oder Nitro ein- oder mehrfach substituiert sein kann und beispielsweise ein Tolyl-, Dinitrophenyl- oder Nitrophenyl-Rest ist; $-O-CO-R^1$; -O-CO-O-Alkyl, insbesondere mit 1 bis 5 C-Atomen im Alkylrest, oder den über ein N-Atom gebundenen Rest eines Azols oder Benzazols mit mindestens 2 N-Atomen im quasi-aromatischen Fünfring.

Die Acylierung wird zweckmäßigerweise in flüssiger Phase in Anwesenheit eines inerten Lösungs-, Dispergier- oder Verdünnungsmittels oder in einem Überschuß des Acylierungsmittels, zweckmäßigerweise unter Rühren, durchgeführt.

Bei der Acylierung beträgt das Molverhältnis zwischen der Verbindung der Formel Ia und dem Acylierungsmittel der Formel III 1 : 1. Zweckmäßigerweise wird das Acylierungsmittel der Formel III in einem geringen molaren Überschuß eingesetzt. Überschüsse von bis zu 30 mol% sind in der Regel ausreichend, d.h. das Molverhältnis zwischen der Verbindung der Formel Ia und dem Acylierungsmittel der Formel III beträgt normalerweise 1 : (1 bis 1,3), vorzugsweise 1: (1 bis 1,2). Falls bei der Acylierungsreaktion eine Säure abgespalten wird, ist der Zusatz eines Säurefängers, wie z.B. eines Alkylihydroxids, wie z.B. Natrium-, Kalium- oder Lithium-hydroxid, eines tertiären organischen Amins, wie z.B. Pyridin oder Triethylamin, eines Alkalicarbonats oder Alkalibicarbonats, wie z.B. Soda oder Natriumbicarbonat, oder eines Alkalisalzes einer schwachen organischen Säure, wie z.B. Natriumacetat, zweckmäßig. Bei der Acylierungsreaktion können auch geeignete Katalysatoren, wie z.B. 4-Dimethylaminopyridin, zugesetzt werden.

Die Acylierung kann prizipiell bei Temperaturen zwischen -10°C und dem Siedepunkt des verwendeten Lösungs-, Dispergier- oder Verdünnungsmittels erfolgen. In vielen Fällen wird die Umsetzung bei 0 bis 50°C, insbesondere bei 0 bis 30°C und vorzugsweise bei Raumtemperatur, durchgeführt.

Die Verbindungen der Formel III sind Acylierungsmittel und stellen somit z.B. dar: für X = Halogen Säurehalogenide bzw. Halogenameisensäureester, von denen Säurechloride und Chlorameisensäureester bevorzugt sind; für -OH Carbonsäuren; für -O-Alkyl und -O-Aryl Ester, von denen die Tolyl-, 2,4-Dinitro- oder 4-Nitrophenylester bevorzugt sind; für $-O-CO-R^1$ Anhydride; für -O-CO-O-Alkyl gemischte Carbonsäure-Kohlensäure-Anhydride; oder heterocyclische Amide oder Azolide, insbesondere von N,N′-Carbonyldiazolen, wie z.B. N,N′-Carbonyldiimidazol, 2,2′-Carbonyl-ditriazol(1,2,3), 1,1′-Carbonyl-ditriazol-(1,2,4), N,N′-Carbonyl-dipyrazol, 2,2′-Carbonyl-ditriazol (vgl. z.B. H.A. Staab, M. Lücking und F.H. Dürr, Chem. Ber. 95, (1962), 1275 ff, H. A. Staab und A. Mannschreck, Chem. Ber. 95, (1962), 1284 ff.; H.A. Staab und W. Rohr, "Synthesen mit heterocyclischen Amiden (Azoliden)" in "Neuere Methoden der Präparativen Organischen Chemie", Band V, Verlag Chemie, 1967, S. 53 ff., insbesondere S. 65 bis 69). Die Acylierungsmittel der Formel III können nach an sich bekannten Verfahren hergestellt werden.

Bei der Verwendung einer Carbonsäure als Acylierungsmittel ist der Zusatz eines Aktivierungsmittels zweckmäßig, das die Aufgabe hat, das Acylierungspotential der Carbonsäure zu erhöhen oder zu aktivieren bzw. die Carbonsäure in situ oder vorzugsweise kurz vor der Umsetzung mit der Verbindung der Formel Ia in ein reaktives Carbonsäurederivat der Formel III zu überführen. Als derartige Aktivierungsmittel sind z.B. geeignet: N,N′-disubstituierte Carbodiimide, insbesondere wenn sie mindestens einen sekundären oder tertiären Alkylrest enthalten, wie z.B. Diisopropyl-, Dicyclohexyl- oder N-Methyl-N′-tert.butylcarbodiimid (vgl. Methodicum Chimicum, Verlag G.Thieme, Stuttgart, Bd. 6, (1974), S.682/683, und Houben-Weyl, Methoden der Org. Chemie, Bd.8, (1952), S. 521/522); Kohlensäurederivate, wie z.B. Phosgen, Chlorameisensäureester, insbesondere mit 1 bis 5 C-Atomen im Alkylrest (vgl. z.B. Tetrahedron Letters 24 (1983), 3365 bis 3368); Kohlensäureester, wie z.B. N,N′-Disuccinimido-carbonat, Diphthalimido-carbonat, 1,1′-(Carbonyldioxy)-dibenzo-triazol oder Di-2-pyridyl-carbonat (vgl. z.B. Tetrahedron Letters, Vol.25, No. 43,

4943-4946), gegebenenfalls in Anwesenheit geeigneter Katalysatoren, wie z.B. 4-Dimethylaminopyridin. Ferner sind als Aktivierungsmittel N,N′-Carbonyldiazole, wie z.B. N,N′-Carbonyl-diimidazol, 2,2′-Carbonyl-ditriazol(1,2,3), 1,1′-Carbonyl-ditriazol(1,2,4), N,N′-Carbonyl-dipyrazol, 2,2′-Carbonyl-ditetrazol, N,N′-Carbonyl-benzimidazol oder N,N′-Carbonylbenztriazol, geeignet (vgl. z.B. H.A. Staab, M. Lücking und F.H. Dürr, loc. cit; H.A. Staab und A. Mannschreck loc. cit.; H.A. Staab und W. Rohr loc. cit). Als N,N′-Carbonyldiazol wird häufig das käufliche N,N′-Carbonyl-diimidazol verwendet. Die anderen N,N′-Carbonylazole sind aber aus dem jeweiligen Azol und Phosgen ebenfalls leicht zugänglich.

Als Aktivierungsmittel für Carbonsäuren sind ferner geeignet: Derivate der Oxalsäure, wie z.B. Oxalylchlorid (vgl. z.B. GB-PS 2 139 225) oder N,N′-Oxalyl-diazole wie z.B. 1,1′-Oxalyldi-imidazol, 1,1′-Oxalyldi-1,2,4-triazol und 1,1′-Oxalyldi-1,2,3,4-tetrazol (vgl. z.B. Shizuaka Murata, Bull.Chem. Soc.Jap. 57, 3597-3598 (1984)); Methylethylphosphinsäureanhydrid (vgl. z.B. DE-OS 31 01 427); Diphosphortetrajodid (Chem. Lett. 1983, 449); Dialkyldisulfit (Indian J. Chem. 21, 259 (1982)); oder andere reaktive Agentien.

Geeignete Lösungs-, Dispergier- oder Verdünnungsmittel sind z.B. solche, die für die Durchführung der Cyclisierung angegeben worden sind, darüber hinaus auch z.B. Pyridin und Amide, wie z.B. Dimethylformamid. Neben Wasser werden für die Acylierung polare organische Lösungsmittel, wie Dimethylformamid, Dimethylsulfoxid oder Pyridin bevorzugt. Auch Lösungsmittelgemische, wie z.B. ein Gemisch aus Wasser und Methylenchlorid, sind geeignet.

Die substituierten 3-Amino-sydnonimine der allgemeinen Formel I bilden mit anorganischen oder organischen Säuren Säureadditionssalze. Zur Bildung derartiger Säureadditionssalze sind anorganische oder organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Pharmakologisch annehmbare Säureadditionssalze werden bevorzugt. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindungen der Formel Ia fallen normalerweise die Säureadditionssalze an. Aus den Säureadditionssalzen können die freien Verbindungen der allgemeinen Formel I bzw. Ia gewünschtenfalls in bekannter Weise, d.h. durch Auflösen oder Suspendieren in Wasser und Alkalischstellen, z.B. mit Natronlauge, und anschließendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel IV

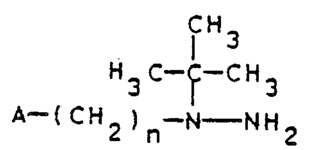

(IV)

worin A und n die bereits genannten Bedeutungen besitzen, durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

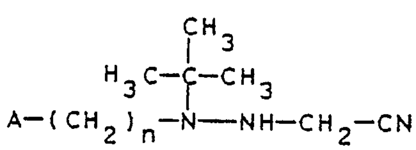

(V)

entsteht, die durch Nitrosierung in die Verbindung II überführt wird. Die Nitrosierung wird in bekannter Weise in einem geeigneten Lösungsmittel, vorzugsweise in Wasser, z.B. bei Temperaturen von 0 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Verbindung V mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Lösung der dabei erhaltenen Verbindung II kann direkt der Cyclisierungsreaktion unterworfen werden. Normalerweise ist es jedoch angebracht, die Nitrosoverbindung II zunächst in einem geeigneten

organischen Lösungsmittel aufzunehmen und in ihm, gegebenenfalls nach Zusatz eines weiteren Lösungsmittels, die Cyclisierung zu der Verbindung der Formel Ia durchzuführen.

Die Verbindungen der allgemeinen Formel IV sind zum Teil bekannt bzw. lassen sich, ausgehend von Verbindungen der allgemeinen Formel VI

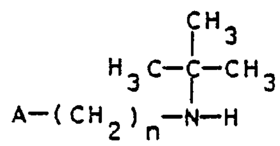

(VI)

dadurch herstellen, daß entweder

a) eine Verbindung der Formel VI zur N-Nitrosoverbindung VII nitrosiert und anschließend, zweckmäßigerweise mit Lithiumaluminiumhydrid, reduziert wird:

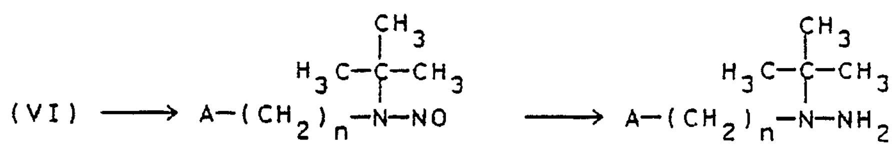

(VII) (IV)

oder daß in an sich bekannter Weise

b) eine Verbindung der Formel VI mit Kaliumcyanat im sauren Medium in das Harnstoffderivat VIII überführt wird, das dann durch Oxydation mit Natriumhypochlorit nach dem Hoffmann-Abbau in die Verbindung IV überführt wird.

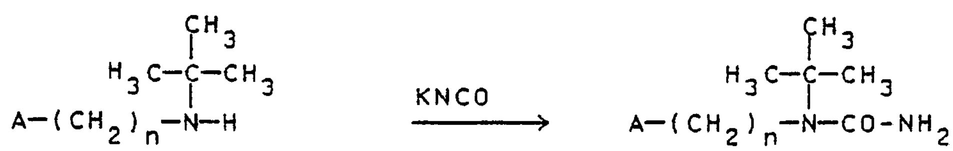


(VI) (VIII)

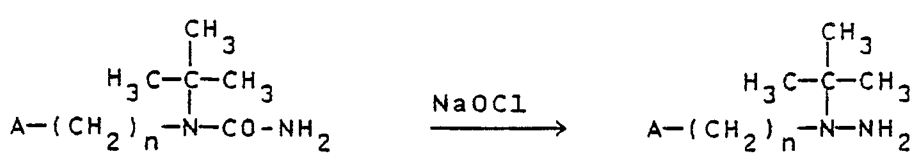


(VIII) (IV)

Die Herstellung der Ausgangsverbindungen der Formeln IV und VI ist bekannt oder kann nach an sich bekannten Verfahren durchgeführt werden.

Die Verbindungen der allgemeinen Formel I und ihre pharmakologisch annehmbaren Säureadditionssalze besitzen wertvolle pharmakologische Eigenschaften. Besonders ausgeprägt ist ihre Wirkung auf das Herz-Kreislauf-System. Verglichen mit bekannten, in 3-Stellung substituierten Sydnoniminverbindungen, z.B. solchen der EP-B-59356, sowie der im Handel befindlichen strukturähnlichen Verbindung Molsidomin, besitzen sie überraschenderweise eine wesentlich längere Wirkungsdauer. Sie senken beispielsweise den Blutdruck ebenso wie den Pulmonalarteriendruck und den linksventrikulären enddiastolischen Druck und

tragen so zu einer Entlastung der Herztätigkeit im Sinne einer antianginösen Wirkung bei, ohne dabei eine reflektorische Tachykardie zu provozieren.

Durch Hemmung der Thrombocytenaggregation können die Verbindungen außerdem antithrombotische Effekte zeigen.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspen sionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblocker, wie z.B. Propra nolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Benzafibrat, Fenofibrat; Mittel für die Thromboseprophylaxe, wie z.B. Phenprocoumon.

Die Verbindungen der Formel I, ihre pharmakologisch annehmbaren Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermit telt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus. Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). In der folgenden Tabelle sind die so erhaltenen $IC_{50}$-Werte angegeben. Wie der Vergleich mit dem $IC_{50}$-Wert $3 \cdot 10^{-4}$ für die bekannte Verbindung Molsidomin (N-Ethoxycarbonyl-3-morpholino-sydnonimin), vgl. DE-B-16 95 897, ergibt, liegen die Werte für die Verbindungen der Formel I erheblich günstiger.

| $IC_{50}$-Werte in mol/l | $IC_{50}$ |
|---|---|
| a) 3-(tert-Butyl-2-hydroxyethyl-amino)-sydnonimin-hydrochlorid | $7 \cdot 10^{-6}$ |
| b) 3-(tert-Butyl-(2-diisopropylaminoethyl)-amino)-sydnonimin-dihydrochlorid | $4 \cdot 10^{-6}$ |
| c) 3-(tert-Butyl-(2-morpholinoethyl)-amino)-sydnonimin-hydrochlorid | $4 \cdot 10^{-6}$ |
| d) N-Ethoxycarbonyl-3-morpholino-sydnonimin | $3 \cdot 10^{-4}$ |
| a bis c: Erfindungsgemäße Verbindungen<br>d: Vergleichsverbindung Molsidomin | |

## Beispiel 1

### 3-(tert-Butyl-2-hydroxyethyl-amino)-sydnonimin-hydrochlorid

#### a) N-Nitroso-tert-butyl-(2-hydroxyethyl)-amin

23,4 g 2-tert.Butylamino-ethanol werden in 60 ml Wasser gelöst und mit 19,7 g konz. Salzsäure versetzt. Die Lösung von 20,7 g Natriumnitrit in 30 ml Wasser wird bei 30-40°C zugetropft. Nach beendeter Zugabe wird die Mischung 1 Stunde auf 70°C erwärmt und dann im Eisbad gerührt. Das Produkt wird abgesaugt bzw. im Falle der öligen Derivate der nachfolgenden Beispiele mit Diethylether ausgeschüttelt und die Ether-Phase getrocknet und eingeengt.
Ausbeute: 23,9 g Fp. 65-68°C

#### b) N-tert-Butyl-N-(2-hydroxyethyl)-hydrazin

Die Mischung von 23 g N-Nitroso-tert.butyl-(2-hydroxyethyl)-amin und 200 ml wasserfreies Tetrahydrofuran wird portionsweise bei 60°C mit insgesamt 12,2 g Lithiumalanat versetzt. Nach beendeter Zugabe wird noch 1 Stunde auf 60°C erwärmt, im Eisbad abgekühlt und das überschüssige Lithiumalanat durch vorsichtiges Zutropfen von Methanol und dann Wasser hydrolysiert. Die Feststoffe werden durch Absaugen entfernt, das Filtrat eingeengt. Es bleibt ein farbloses Öl (14 g), das ohne weitere Reinigung weiterverwendet wird.

#### c) 3-(tert-Butyl-(2-hydroxyethyl)-amino)-sydnoniminhydrochlorid

Das in der Stufe b erhaltene Öl (14 g) wird in 50 ml Wasser und 8,1 g konz. Salzsäure gelöst und auf -5°C abgekühlt. Dann wird die Lösung von 6,6 g Kaliumcyanid in 20 ml Wasser zugetropft. Nach Zugabe von 15 ml Ethanol wird eine 39%ige Formalinlösung (8,2 g) zugetropft und der pH-Wert der Mischung auf 7 eingestellt. Es wird 2 Stunden bei Raumtemperatur gerührt, mit konz. Salzsäure auf pH = 1,5 gestellt und unter Eiskühlung die Lösung von 5,6 g Natriumnitrit in 25 ml Wasser zugetropft. Es wird über Nacht bei Raumtemperatur nachgerührt und dann mit Diethylether extrahiert, getrocknet und eingeengt. Das zurückbleibende Öl wird in 50 ml konz. methanolische Salzsäure gelöst und mit 50 ml Essigsäureethylester verdünnt. Der ausgefallene Niederschlag wird abgesaugt und verworfen. Das Filtrat wird nach 2 Stunden eingeengt und der Rückstand mit Essigsäureethylester verrührt und der Feststoff abgesaugt und aus Ethanol/Diethylether (1:2) umkristallisiert.
Ausbeute: 5,9 g Fp = 161-163°C (Zers.)

## Beispiel 2

N-Ethoxycarbonyl-3-(tert-Butyl-2-hydroxy-ethyl-amino)-sydnonimin

5,7 g 3-(tert-Butyl-2-hydroxyethyl-amino)-sydnoniminhydrochlorid gemäß Beispiel 1 c werden in 20 ml Wasser gelöst und auf 0 bis 5°C abgekühlt und mit 5,1 g wasserfreiem Natriumbicarbonat versetzt. Darauf wird die Lösung von 3,9 g Chlorameisensäureethylester in 30 ml Methylenchlorid zugetropft. Nach beendeter Zugabe wird 1 Stunde nachgerührt, die Methylenchlorid-Phase abgetrennt, getrocknet und eingeengt. Der Rückstand wird aus Essigsäureisopropylester umkristallisiert.
Fp.: 111-113°C

Analog Beispiel 1 werden hergestellt:

Beispiel 3

3-(tert-Butyl-(2-methoxyethyl)-amino)-sydnonimin-hydrochlorid

a) N-Nitroso-tert-butyl-(2-methoxyethyl)-amin (gelbes Öl)
b) N-tert-Butyl-N-(2-methoxyethyl)-hydrazin (Öl)
c) 3-(tert-Butyl-(2-methoxyethyl)-amino-sydnonimin-hydrochlorid
Fp. : 147°C (Zers.)

Beispiel 4

3-tert-Butyl-(2-isopropoxyethyl-amino)-sydnonimin-hydrochlorid

a) N-Nitroso-tert-butyl-(2-isopropoxyethyl)-amin (gelbes Öl)
b) N-tert-Butyl-N-(2-isopropoxyethyl)-hydrazin (Öl)
c) 3-(tert-Butyl-(2-isopropoxyethyl)amino-sydnonimin-hydrochlorid
Fp. : 160°C (Zers.)

Beispiel 5

3-(tert-Butyl-(2-dimethylaminoethyl-amino)-sydnonimin-dihydrochlorid

a) N-Nitroso-tert-butyl-(2-dimethylamino-ethyl)-amin (gelbes Öl)
b) N-tert-Butyl-N-(2-dimethylamino-ethyl)-hydrazin (Öl)
c) 3-(tert-Butyl-(2-dimethylaminoethyl)-amino)-sydnonimin-dihydrochlorid
Fp.: 133°C (Zers.)

Beispiel 6

3-(tert-Butyl-(2-diisopropylaminoethyl)-amino)-sydnonimin-dihydrochlorid

a) N-Nitroso-tert-Butyl-(2-diisopropylaminoethyl)-amin (gelbes Öl)
b) N-tert-Butyl-N-(2-diisopropylaminoethyl)-hydrazin (Öl)
c) 3-(tert-Butyl-(2-diisopropylaminoethyl)-amino)-sydnonimin-dihydrochlorid
Fp. : 143°C (Zers.)

Beispiel 7

3-(tert-Butyl-(2-morpholinoethyl-amino)-sydnonimin-dihydrochlorid

FP.: 159 - 160°C (Zers.)

Analog Beispiel 2 werden hergestellt:

Beispiel 8

N-Benzoyl-3-(tert-butyl-2-methoxyethyl)-amino)-sydnonimin

FP.: 122 - 124°C

Beispiel 9

N-Cyclohexylcarbonyl-3-(tert-butyl-2-isopropoxyethylamino-sydnonimin

FP.: 101 - 104°C

Beispiel 10

N-Acetyl-3-(tert-butyl-2-dimethylaminoethyl-amino)-sydnonimin

FP.: 89 - 90°C

Beispiel 11

N-Pivaloyl-3-(tert-butyl-2-diisopropylamino-ethyl-amino)-sydnonimin

FP.: 90 - 92°C

In den nachfolgenden Beispielen A - F werden pharmazeutische Präparate beschrieben.

Beispiel A

| Gelatineweichkapseln, enthaltend 5 mg Wirkstoff pro Kapsel: | |
|---|---|
| | pro Kapsel |
| Wirkstoff | 5 mg |
| Aus Kokosfett fraktioniertes Tri glycerid-Gemisch | 150 mg |
| Kapselinhalt | 155 mg |

Beispiel B

| Injektionslösung, enthaltend 1 mg Wirkstoff pro ml: | |
|---|---|
| | pro ml |
| Wirkstoff | 1,0 mg |
| Polyethylenglycol 400 | 0,3 ml |
| Natriumchlorid | 2,7 mg |
| Wasser zu Injektionszwecken | ad 1 ml |

Beispiel C

| Emulsion, enthaltend 3 mg Wirkstoff pro 5 ml | |
|---|---|
| | pro 100 ml Emulsion |
| Wirkstoff | 0,06 g |
| Neutralöl | q.s. |
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

Beispiel D

| Rektale Arzneiform, enthaltend 4 mg Wirkstoff pro Suppositorium | |
|---|---|
| | pro Suppositorium |
| Wirkstoff | 4 mg |
| Suppositoriengrundmasse | ad 2 g |

Beispiel E

| Tabletten, enthaltend 2 mg Wirkstoff pro Tablette | |
|---|---|
| | pro Tablette |
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel F

| Dragees, enthaltend 1 mg Wirkstoff pro Dragee | |
|---|---|
| | pro Dragee |
| Wirkstoff | 1 mg |
| Maisstärke | 100 mg |
| Lactose | 60 mg |
| sec Calciumphosphat | 30 mg |
| lösliche Stärke | 3 mg |
| Magnesiumstearat | 2 mg |
| kolloidale Kieselsäure | 4 mg |
| | 200 mg |

## Ansprüche

1. Substituierte 3-Aminosydnonimine der allgemeinen Formel I

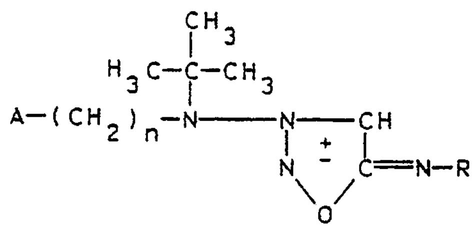

(I)

und ihre pharmakologisch annehmbaren Säureadditionssalze, worin
A Hydroxy; Alkoxy mit 1 bis 4 C-Atomen; Dialkylamino mit insgesamt 2 bis 8 C-Atomen; Morpholino; Piperidino; Pyrrolidin-1-yl;
R Wasserstoff oder den Rest $-COR^1$;
$R^1$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit insgesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 2 oder 3
bedeuten.

2. Substituierte 3-Aminosydnonimine nach Anspruch 1, dadurch gekennzeichnet, daß n die Zahl 2 bedeutet.

3. Substituierte 3-Aminosydnonimine nach Anspruch 1 und/oder 2, dadurch gekennzeichnet, daß A Hydroxy, Methoxy, Ethoxy, Isopropoxy, Dimethylamino, Diethylamino, Di-n-propylamino, Diisopropylamino oder Morpholino bedeutet.

4. Substituierte 3-Aminosydnonime nach einem oder mehreren der Ansprüche 1 bis 3, dadurch gekennzeichnet, daß R Wasserstoff oder $-COR^1$ mit $R^1$ = Methyl, Ethyl, Propyl, Isopropyl, tert-Butyl, Cyclohexyl, Methoxy, Ethoxy, Isopropoxy, Butoxy oder Phenyl bedeutet.

5. 3-(tert-Butyl-2-hydroxyethyl-amino)-sydnonimin und seine pharmakologisch annehmbaren Säureadditionssalze.

6. 3-(tert-Butyl-2-diisopropylaminoethyl-amino)-sydnonimin und seine pharmakologisch annehmbaren Säureadditionssalze.

7. Verfahren zur Herstellung der in einem oder mehreren der Ansprüche 1 bis 3 angegebenen Verbindungen der Formel I oder der in den Ansprüchen 5 und 6 angegebenen Verbindungen, dadurch gekennzeichnet, daß eine Verbindung der allgemeinen Formel II

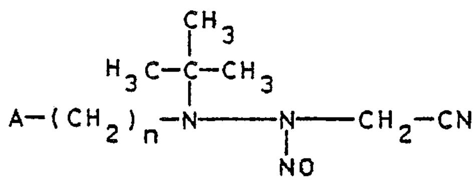

(II)

worin A und n die in einem oder mehreren der Ansprüche 1 bis 3 angegebenen Bedeutungen besitzen, zu einer Verbindung der Formel Ia

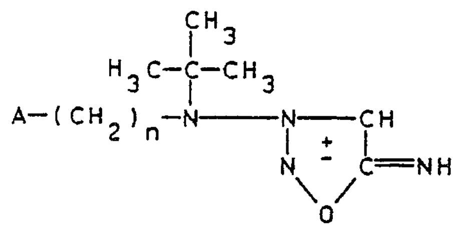

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit R = $-COR^1$ hergestellt wird, die Verbindung der Formel Ia oder ein Säureadditionssalz davon mit einem Acylierungsmittel acyliert wird, das den Rest $-COR^1$ einführt und die erhaltene Verbindung gegebenenfalls in ein Säureadditionssalz überführt wird.

8. Verfahren nach Anspruch 7, dadurch gekennzeichnet, daß die Cyclisierung in einem Lösungs-, Dispergier- oder Verdünnungsmittel bei Temperaturen von -10 bis 40°C, vorzugsweise 0 bis 20°C, mit Hilfe eines Cyclisierungsmittels, das in wäßriger Lösung einen pH-Wert unter 3 einstellt, durchgeführt wird.

9. Verwendung eines 3-Amino-sydnonimins der Ansprüche 1 bis 6 und/oder eines seiner pharmakologisch annehmbaren Säureadditionssalze als pharmakologischer Wirkstoff zur Herstellung pharmazeutischer Präparate.

10. Pharmazeutisches Präparat, dadurch gekennzeichnet, daß es eine Verbindung der Ansprüche 1 bis 6 oder ein Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

Patentansprüche für folgenden Vertragsstaat: ES

1. Verfahren zur Herstellung von substituierten 3-Aminosydnoniminen der allgemeinen Formel I

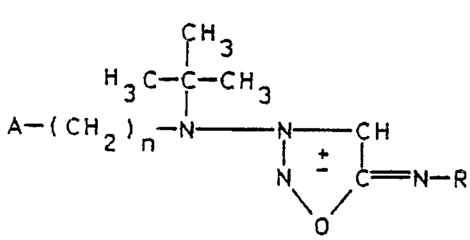

(I)

und ihrer pharmakologisch annehmbaren Säureadditionssalze, worin

A Hydroxy; Alkoxy mit 1 bis 4 C-Atomen; Dialkylamino mit insgesamt 2 bis 8 C-Atomen; Morpholino; Piperidino; Pyrrolidin-1-yl;

R Wasserstoff oder den Rest $-COR^1$;

$R^1$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit ins gesamt 2 bis 7 C-Atomen; einen Arylrest mit 6 bis 10 C-Atomen; einen durch 1 bis 3 Halogenatome und/oder 1 bis 3 Alkylreste mit 1 bis 3

C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 2 oder 3
bedeuten, dadurch gekennzeichnet, daß eine Verbindung der allgemeinen Formel II

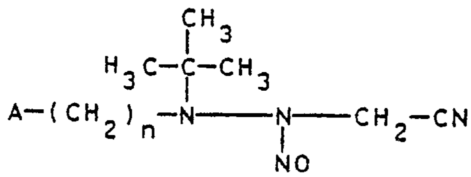

(II)

worin A und n die angegebenen Bedeutungen besitzen, zu einer Verbindung der Formel Ia

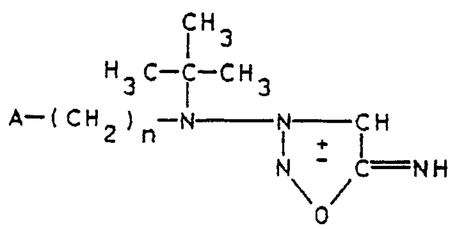

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit R = -COR[1] hergestellt wird, die Verbindung der Formel Ia oder ein Säureadditionssalz davon mit einem Acylierungsmittel acyliert wird, das den Rest -COR[1] einführt und die erhaltene Verbindung gegebenenfalls in ein Säureadditionssalz überführt wird.

2. Verfahren nach Anspruch 1, dadurch gekennzeichnet, daß eine Verbindung der Formel II eingesetzt wird, bei der n die Zahl 2 bedeutet.

3. Verfahren nach Anspruch 1 und/oder 2, dadurch gekennzeichnet, daß eine Verbindung der Formel II eingesetzt wird, bei der A Hydroxy, Methoxy, Ethoxy, Isopropoxy, Dimethylamino, Diethylamino, Di-n-propylamino, Di-isopropylamino oder Morpholino bedeutet.

4. Verfahren nach einem oder mehreren der Ansprüche 1 bis 3, dadurch gekennzeichnet, daß nach der Cyclisierung keine Acylierung durchgeführt wird.

5. Verfahren nach einem oder mehreren der Ansprüche 1 bis 3, dadurch gekennzeichnet, daß bei der Acylierung ein Acylierungsmittel eingesetzt wird, das den Rest -COR[1] einführt, wobei R[1] = Methyl, Ethyl, Propyl, Isopropyl, tert-Butyl, Cyclohexyl, Methoxy, Ethoxy, Isopropoxy, Butoxy oder Phenyl bedeutet.

6. Verfahren nach einem oder mehreren der Ansprüche 1 bis 4, dadurch gekennzeichnet, daß die Ausgangsverbindungen so ausgewählt werden, daß das 3-(tert-Butyl-2-hydroxyethyl-amino)-sydnonimin oder eines seiner pharmakologisch annehmbaren Säureadditionssalze entsteht.

7. Verfahren nach einem oder mehreren der Ansprüche 1 bis 4, dadurch gekennzeichnet, daß die Ausgangsverbindungen so ausgewählt werden, daß das 3-(tert-Butyl-2-diisopropylaminoethyl-amino)-sydnonimin oder eines seiner pharmakologisch annehmbaren Säureadditionssalze entsteht.

8. Verfahren nach einem oder mehreren der Ansprüche 1 bis 7, dadurch gekennzeichnet, daß die Cyclisierung in einem Lösungs-, Dispergier- oder Verdünnungsmittel bei Temperaturen von -10 bis 40°C, vorzugsweise 0 bis 20°C, durchgeführt wird.

9. Verfahren nach einem oder mehreren der Ansprüche 1 bis 8, dadurch gekennzeichnet, daß die Cyclisierung mit Hilfe eines Cyclisierungsmittels, das in wäßriger Lösung einen pH-Wert unter 3 einstellt, durchgeführt wird.

10. Verwendung eines substituierten 3-Aminosydnonimins der allgemeinen Formel I

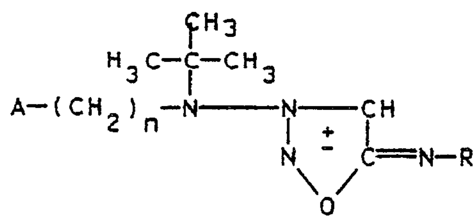

(I)

und/oder eines seiner pharmakologisch annehmbaren Säureadditionssalze, worin
A Hydroxy; Alkoxy mit 1 bis 4 C-Atomen; Dialkylamino mit insgesamt 2 bis 8 C-Atomen; Morpholino; Piperidino; Pyrrolidin-1-yl;
R Wasserstoff oder den Rest $-COR^1$;
$R^1$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit ins gesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 2 oder 3
bedeuten, als pharmakologischer Wirkstoff zur Herstellung pharmazeutischer Präparate.

Patentansprüche für folgenden Vertragsstaat: GR

1. Substituierte 3-Aminosydnonimine der allgemeinen Formel I

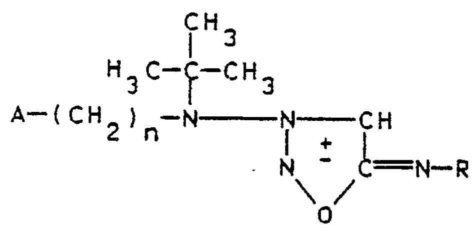

(I)

und ihre pharmakologisch annehmbaren Säureadditionssalze, worin
A Hydroxy; Alkoxy mit 1 bis 4 C-Atomen; Dialkylamino mit insgesamt 2 bis 8 C-Atomen; Morpholino; Piperidino; Pyrrolidin-1-yl;
R Wasserstoff oder den Rest $-COR^1$;
$R^1$ einen aliphatischen Rest mit 1 bis 4 C-Atomen, der auch durch Alkoxy mit 1 bis 3 C-Atomen substituiert sein kann; einen cycloaliphatischen Rest mit 5 bis 7 C-Atomen; einen bicycloaliphatischen Rest mit 7 bis 14 C-Atomen; einen tricycloaliphatischen Rest mit 7 bis 16 C-Atomen; einen Alkoxyrest mit 1 bis 6 C-Atomen; einen Aryloxyrest mit 6 bis 10 C-Atomen; einen Alkoxycarbonylrest mit ins gesamt 2 bis 7 C-Atomen; einen Arylrest mit 6 bis 10 C-Atomen; einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 10 C-Atomen;
n die Zahl 2 oder 3
bedeuten.

2. Substituierte 3-Aminosydnonimine nach Anspruch 1, dadurch gekennzeichnet, daß n die Zahl 2 bedeutet.

3. Substituierte 3-Aminosydnonimine nach Anspruch 1 und/oder 2, dadurch gekennzeichnet, daß A Hydroxy, Methoxy, Ethoxy, Isopropoxy, Dimethylamino, Diethylamino, Di-n-propylamino, Diisopropylamino oder Morpholino bedeutet.

4. Substituierte 3-Aminosydnonime nach einem oder mehreren der Ansprüche 1 bis 3, dadurch gekennzeichnet, daß R Wasserstoff oder $-COR^1$ mit $R^1$ = Methyl, Ethyl, Propyl, Isopropyl, tert-Butyl, Cyclohexyl, Methoxy, Ethoxy, Isopropoxy, Butoxy oder Phenyl bedeutet.

5. 3-(tert-Butyl-2-hydroxyethyl-amino)-sydnonimin und seine pharmakologisch annehmbaren Säureadditionssalze.

6. 3-(tert-Butyl-2-diisopropylaminoethyl-amino)-sydnonimin und seine pharmakologisch annehmbaren Säureadditionssalze.

7. Verfahren zur Herstellung der in einem oder mehreren der Ansprüche 1 bis 3 angegebenen Verbindungen der Formel I oder der in den Ansprüchen 5 und 6 angegebenen Verbindungen, dadurch gekennzeichnet, daß eine Verbindung der allgemeinen Formel II

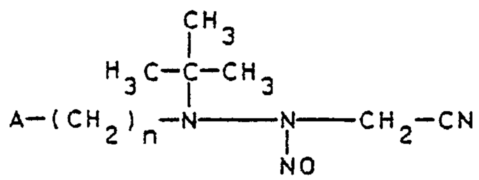

(II)

worin A und n die in einem oder mehreren der Ansprüche 1 bis 3 angegebenen Bedeutungen besitzen, zu einer Verbindung der Formel Ia

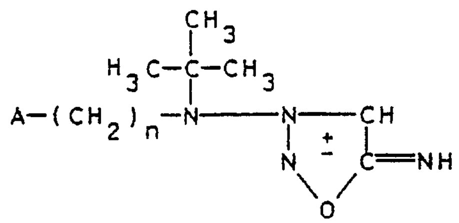

(Ia)

die auch in Form eines Säureadditionssalzes vorliegen kann, cyclisiert wird und gegebenenfalls aus dem Säureadditionssalz die freie Verbindung isoliert wird und daß für den Fall, daß eine Verbindung der Formel I mit R = $-COR^1$ hergestellt wird, die Verbindung der Formel Ia oder ein Säureadditionssalze davon mit einem Acylierungsmittel acyliert wird, das den Rest $-COR^1$ einführt und die erhaltene Verbindung gegebenenfalls in ein Säureadditionssalz überführt wird.

8. Verfahren nach Anspruch 7, dadurch gekennzeichnet, daß die Cyclisierung in einem Lösungs-, Dispergier- oder Verdünnungsmittel bei Temperaturen von -10 bis 40°C, vorzugsweise 0 bis 20°C, durchgeführt wird.

9. Verfahren nach Anspruch 7 und/oder 8, dadurch gekennzeichnet, daß die Cyclisierung mit Hilfe eines Cyclisierungsmittels, das in wäßriger Lösung einen pH-Wert unter 3 einstellt, durchgeführt wird.

10. Verwendung eines 3-Amino-sydnonimins der Ansprüche 1 bis 6 und/oder eines seiner pharmakologisch annehmbaren Säureadditionssalze als pharmakologischer Wirkstoff zur Herstellung von pharmazeutischen Präparaten.

Europäisches Patentamt

# EUROPÄISCHER RECHERCHENBERICHT

Nummer der Anmeldung

EP 89109809.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | EP - A1 - 0 210 474 (CASSELLA) * Formel I; Ansprüche 9,10 * -- | 1,9,10 | C 07 D 271/04<br>A 61 K 31/41 |
| A | DE - A1 - 1 620 501 (TAKEDA) * Seite 1, Zeile 1 - Seite 2, Zeile 3 * -- | 1,9,10 | |
| A | AT - B - 275 514 (BOEHRINGER) * Anspruch 1 * -- | 1 | |
| A | AT - B - 287 710 (BOEHRINGER) * Anspruch 1 * -- | 1 | |
| A | CHEMICAL ABSTRACTS, Band 106, Nr. 9, 2. März 1987, Columbus, Ohio, USA VEDRILLA VEIT, DAGMAR et al. "Substituted sydnonimines" Seite 615, Spalte 1, Zusammenfassung-Nr. 67 327n & Span. ES 547,311 -- | 1 | RECHERCHIERTE SACHGEBIETE (Int. Cl.4)<br>C 07 D 271/00 |
| A | CHEMICAL ABSTRACTS, Band 107, Nr. 1, 6. Juli 1987, Columbus, Ohio, USA VILA CASAS, ANTONIO et al. "Process for the preparation of N-acyl-3-morpholinosydnone imines" Seite 666, Spalte 2, Zusammenfassung-Nr. 7 201c & Span. ES 547,310 ---- | 1 | |

Der vorliegende Recherchenbericht wurde für alle Patentansprüche erstellt.

| Recherchenort | Abschlußdatum der Recherche | Prüfer |
|---|---|---|
| WIEN | 01-09-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument